# EUROPEAN PATENT SPECIFICATION

(11) **EP 2 550 990 B1**
(45) Date of publication and mention of the grant of the patent: **06.05.2020**
(21) Application number: 11758801.2
(22) Date of filing: 23.03.2011
(51) Int. Cl.: A61M 27/00, A61M 1/00

(54) **MEDICAL VACUUM SEALING DRAINAGE DEVICE**
MEDIZINISCHE DRAINAGEVORRICHTUNG FÜR VAKUUMVERSIEGELUNG
DISPOSITIF DE DRAINAGE ÉTANCHÉIFIANT SOUS VIDE MÉDICAL

(30) Priority: 10.01.2011 CN 201120005386 U; 29.06.2010 CN 201020252540 U; 29.06.2010 CN 201010221569; 23.03.2010 CN 201010132585
(43) Date of publication of application: 30.01.2013
(73) Proprietor: Wuhan Vsd Medical Science&Technology Co. Ltd., Wuhan, Hubei 430022 (CN)
(72) Inventor: SONG, Jiuhong, Wuhan Hubei 430022 (CN)
(74) Representative: Zeuner Summerer Stütz
(86) International application number: PCT/CN2011/072066
(87) International publication number: WO 2011/116691

(56) References cited:
- WO-A2-03/057070
- WO-A2-2007/006306
- WO-A2-2008/004161
- CN-A- 101 217 994
- CN-A- 101 390 790
- CN-A- 101 474 432
- CN-A- 101 829 388
- CN-A- 101 904 768
- CN-U- 201 529 311
- CN-U- 201 744 062
- CN-Y- 2 453 904
- CN-Y- 2 698 359
- JP-A- 2006 223 623
- JP-A- 2006 223 623
- US-A1- 2005 137 539
- US-A1- 2007 027 414
- US-A1- 2009 318 842

## Description

### FIELD OF THE INVENTION

The invention relates to a medical vacuum sealing drainage device, which is suitable for drainage of wounds on the body surface and for vacuum sealing drainage in a body cavity.

### BACKGROUND OF THE INVENTION

A vacuum sealing drainage technology (VSD for short) was invented by Dr. Wim Fleischman at the University of Ulm Germany in 1992 and theory thereof has been formed, and it is for drainage of wound surface of limbs. In 1994, Chinese professor Qiu Huade first applied the VSD technology to general surgical departments, and initiated application of the VSD technology in the general surgical departments, and initiated application of the VSD technology in the general surgical departments. Professors Wang Yanfeng and Qiu Huade got the first patent of the VSD technology with a patent number CN 2236350 on March 17, 1997.

A basic configuration of the conventional VSD technology includes a vacuum source (including a medical suction unit, a central vacuum unit, or a vacuum drainage bottle used in a hospital), a drainage pipe, polyvinyl alcohol foams orother medical multi-hole foams, sponges or gauzes (a multi-hole foam cushion for short), a breathable film for adhesion and sealing (a sealing film for short), and a drainage container. The vacuum source is connected for drainage, whereby conducting liquefaction materials of body tissues such as exudation, cataclysm, liquefaction necrosis tissue fragments, pus and so on into the drainage container.

In use, the drainage pipe with multiple side holes is wrapped with the multi-hole foam cushion, and disposed on the wound surface or in a wound cavity. The sealing film is used to tightly seal the multi-hole foam cushion and an outlet of the drainage pipe whereby separating them from the outside. Then the drainage container is connected, and finally the vacuum source is connected, and thus a drainage system is formed. Since vesicles in the multi-hole foam cushion are connected to each other and are rich in flexibility, negative pressure can reach every point of a targeted drainage area whereby forming omnibearing drainage. Under the action of the negative pressure, comparatively big, tender and blocky educts are cut and molded into granules, which enter the drainage pipe via openings of the multi-hole foam cushion or vesicles connected to each other, and then are quickly inhaled into the drainage container. Big educts that may block the drainage pipe are stopped by the multi-hole foam cushion, adhered to the surface of the multi-hole foam cushion, and left a body of the device along with the multi-hole foam cushion as drain is removed or replaced. Closing of the sealing film is a critical factor to maintain negative pressure operating as drainage force, and to separate the drainage area from the outside, which effectively prevent pollution and cross infection.

However, during use of the conventional VSD technology in drainage of a surface wound tissue, several problems exist: 1) sealing thereof is inconvenient. Good sealing is a key for ensuring drainage effect and the most difficult step during the whole vacuum sealing drainage. Common-used sealing methods include: a method of stabbing holes, a mesentery method, a suture method, a dumpling method, and so on. However, the above-mentioned methods are very complex during practical operation, and operative doctors can grasp them after repeated operations and practices. In addition, infirm sealing often causes air leakage, and further decreasing of treatment effect or even treatment failure of the VSD system. 2) VSD materials would be dehydrated and become dried and hard, if being under a negative pressure for a long time. 3) Sealing cannot be achieved, when the system is used for the drainage of orthopedics or perineum.
Document US 2007/027414 discloses a vacuum assisted would treatment device with a porous foam soft pad. WO 2007/006306 A2 discloses an access port for enabling passage of a conduit through a partition wall. Documents US2005/137539 A1, WO 03/057070 A2, WO2008/004161 A2 and JP 2006 223623 A disclose background art.

### SUMMARY OF THE INVENTION

The invention provides a medical vacuum sealing drainage device according to claim 1, an orthopedic vacuum sealing drainage device according to claim 10 and a vacuum sealing drainage device suitable for use at the peritoneum according to claim 13. Preferred embodiments are defined in the dependent claims. The medical vacuum sealing drainage device is good in sealing effect and simple in structure, convenient to use, and not only suitable for drainage of wounds on the body surface, but also suitable for vacuum sealing drainage in a body cavity.

The technical scheme of the invention is as follows: a medical vacuum sealing drainage device, comprises: a porous foam soft pad, and a drainage tube arranged in the porous foam soft pad and provided with a tail end extending outside the porous foam soft pad. An elastic sealing rubber film is arranged on an outer side of the porous foam soft pad; a sealing opening for allowing the drainage tube to extend out is formed on the elastic sealing rubber film; a diameter of an opening part of the sealing opening is less than an outer diameter of a corresponding extending pipe.

The sealing opening used for allowing the drainage tube to extend out and formed on the elastic sealing rubber film is a circle hole, the diameter of which is 0.3-4 mm less than the outer diameter of the corresponding extending pipe.

The sealing opening used for allowing the drainage tube to extend out and formed on the elastic sealing rubber film is a pipe orifice, and the diameter of the opening part of the pipe orifice is 0.3-2 mm less than the outer diameter of the corresponding extending pipe.

When the sealing opening used for allowing tubes to extend out and formed on the elastic sealing rubber film is a pipe orifice, the pipe orifice takes a shape of an inverted horn, the diameter of the opening part of the pipe orifice is 1-2 mm less than the outer diameter of the drainage tube, and the diameter of the bottom of the pipe orifice is 2-6 mm more than the outer diameter of the drainage tube.

When the sealing opening used for allowing tubes to extend out and formed on the elastic sealing rubber film is a pipe orifice, an included angle between the sealing opening and a main body of the elastic sealing rubber film is 15-90°.

A plurality of small holes is formed on the side wall of the drainage tube.

A single or a plurality of drainage tubes is employed; the plurality of drainage tubes are arranged evenly with a distance of 25-35 mm between each other, and connected through a multi-way connector in the tail end.

A flushing pipe is arranged in the drainage tube, one end of the flushing pipe extends to the inside of the drainage tube, the other end of the flushing pipe is led out from the side wall at the tail end of the drainage tube, and a sealing cover is arranged at the tail end of the flushing pipe.

An orthopedic vacuum sealing drainage device, comprises: a porous foam soft pad, and a drainage tube arranged in the porous foam soft pad and provided with a tail end extending outside the porous foam soft pad. A first elastic sealing rubber film is arranged on the outer side of the porous foam soft pad, a sealing opening for allowing the drainage tube to extend out is formed on the first elastic sealing rubber film, the diameter of the opening part of the sealing opening is smaller than the outer diameter of the corresponding drainage tube; a second elastic sealing rubber film and a third elastic sealing rubber film are arranged at puncture needles connected with an orthopedics external fixation device, vertical pipe orifice shaped sealing openings are formed on the second and third elastic sealing rubber films, and sleeved on the two puncture needles respectively; the second and the third elastic sealing rubber films are placed on the first elastic sealing rubber film or directly placed on the porous foam soft pad for sealing in a covering manner.

When the sealing opening used for allowing the drainage tube to extend out and formed on the first elastic sealing rubber films is a pipe orifice, an included angle between the pipe orifice and a main body of the elastic sealing rubber film is 15-90°.

The vertical pipe orifice shaped sealing opening on the second and the third elastic sealing rubber films takes a shape of an inverted horn, the diameter of the opening part of the pipe orifice is 0.3-2 mm less than the outer diameter of the puncture needles, and the diameter of the bottom of the pipe orifice is 2-6 mm more than the outer diameter of the corresponding puncture needles.

A flushing pipe is arranged in the drainage tube, one end of the flushing pipe extends to the inside of the drainage tube, the other end of the flushing pipe is led out from the side wall at the tail end of the drainage tube, and a sealing cover is arranged at the tail end of the sealing cover.

A vacuum sealing drainage device used at the perineum, comprises a porous foam soft pad, and a drainage tube arranged in the porous foam soft pad and provided with a tail end extending outside the porous foam soft pad. A first elastic sealing rubber film is arranged on the outer side of the porous foam soft pad, a sealing opening for allowing the drainage tube to extend out is formed on the first elastic sealing rubber film, and the diameter of the opening part of the sealing opening is smaller than the outer diameter of the corresponding drainage tube; the vacuum sealing drainage device used at the perineum further comprises an anus inserting catheter, a fourth elastic sealing rubber film is arranged at the position where the anus inserting catheter penetrates a vacuum area of the vacuum sealing drainage device, and provided with a vertical pipe orifice shaped sealing opening, the vertical pipe orifice shaped sealing opening is sleeved on the anus inserting catheter, and the fourth elastic sealing rubber film is placed on the first elastic sealing rubber film or directly placed on the porous foam soft pad for sealing in a covering manner.

An annular rubber air-filled or liquid-filled bag is arranged on the outer side at the front end of the anus inserting catheter; an air or liquid guide pipe parallel to the anus inserting catheter is also arranged; the front end of the air or liquid guide pipe is communicated with the rubber air-filled or liquid-filled bag, the rear end of the air or liquid guide pipe extends outside the rear end of the anus inserting catheter, and a sealing cover is arranged at the rear end of the air or liquid guide pipe.

A flushing pipe is arranged in the drainage tube, one end of the flushing pipe extends to the inside of the drainage tube, the other end of the flushing pipe is led out from the side wall at the tail end of the drainage tube, and a sealing cover is arranged at the tail end of the flushing pipe.

Advantages of the invention are summarized below:
(1) The problem of air leakage of the extending part of the drainage tube is solved thoroughly, the stable and successful vacuum sealing drainage is ensured, the key technology for vacuum drainage is overcome, the using effect is better, and the possibility of air leakage of the VSD (vacuum sealing drainage) system is greatly reduced, that is, the conventional air leakage rate is reduced to 5% from 60%.
(2) Due to the arrangement of the elastic sealing rubber film, the device is suitable for drainage of wounds both on the body surface and in the body cavity. Two sets of different conventional drainage devices (drainage of wounds both on the body surface and in the body cavity) are combined into a set.
(3) The operation is more convenient, for the wounds both in the body cavity and on the body surface, the extending part of the drainage tube is sealed through the elastic sealing rubber film, thus the sealing of the sealing film is more simple and easier; the defects of complex structure and heavy load of doctors due to the adoption of the VSD technology for the wounds in the body cavity are overcome. During the drainage process of wounds in the body cavity, an effective support (carrier) is provided for the sealing film, so that the embarrassment that in the radical mastectomy, Miles operation for rectal cancer, radical operation for vaginal tumor, osteomyelitis fenestration, lavage and drainage operation and perineum explosion injury operation, the drainage tube is not provided with a support during the film sticking process, so an opposite sticking method has to be adopted is avoided. The sealing film is only required to be sealed around the elastic sealing rubber film, thus the drainage operation for the wounds in the body cavity is simple, and the sealing effect is good. The complexity to operators during the use of the film sticking method and the harm of the hole poking method to the normal body tissue of patients are avoided; the oppression of the normal tissues around during the drainage at the side outlet hole is avoided.
(4) During the drainage of wounds on the body surface, besides providing a sticking film supporting surface, the elastic sealing rubber film also can covers the porous foam soft pad by increasing its area, so that the problem that the if sealing film is directly stuck on the porous foam soft pad, the porous foam soft pad may become hard due to the evaporation of water in the porous foam soft pad through the sealing film, and then the vacuum sealing drainage will fail is avoided.
(5) The drainage tubes are connected with the catheter through the multi-way connector, the absorption area is increased through a plurality of rows of small holes on the side wall of the drainage tube, the outlet of each drainage tube can be used as both a drainage opening and a flushing opening for flushing a blocked pipe, and both the drainage and flushing are convenient.
(6) The dedicated flushing pipe is used for flushing the blocked pipe, thus the retrograde infection is avoided; the dedicated flushing pipe also can be used for diluting, soaking and softening the drainage in the porous foam soft pad in due time, thus the drainage is more convenient; the operation time is reduced, the pain of patients is alleviated, and the wound healing time of patients is shortened.
(7) The orthopaedic vacuum sealing drainage device changes the operating instructions and rules of the traditional orthopaedic external fixation technology and combines the sealing and fixing operation, the orthopaedic external fixation device is hermetically sealed, so that the problem of failure or reduced effect in vacuum sealing drainage treatment due to air leakage is eliminated, the infection at the puncture needle is also eliminated, the key technology of applying the vacuum sealing drainage device to orthopaedic trauma is overcome, the infection at the orthopaedic trauma puncture needle is basically eliminated, the frequent condition that infection extends to the bone cavity along the puncture needle is completely avoided, the risk of treatment of patients is greatly reduced, the pain of patients is eased, the treatment effect is effectively ensured, and the disability rate of patients is greatly reduced.
(8) According to the vacuum sealing drainage device used at the perineum, because the inserting catheter for the perineum is additionally arranged, in the VSD (vacuum sealing drainage) treatment, the normal physiological excretion of patients is not affected, the excrement is effectively prevented from overflowing to pollute the wound, the incidence rate of infection is effectively lowered, and the wound healing time of patients is reduced.

### BRIEF DESCRIPTION OF THE DRAWINGS

FIG 1 is an overall structure diagram of a first basic vacuum sealing drainage device;
FIG. 2 is an overall structure diagram of a second basic vacuum sealing drainage device;
FIG 3 is a diagram of a medical vacuum sealing drainage device used inside the body;
FIG. **4** is a diagram of a sealing opening of an elastic sealing rubber film taking a shape of a circular hole;
FIG **5** is a diagram of a sealing opening of an elastic sealing rubber film taking a shape of a pipe orifice;
FIG **6** is a structure diagram taken from line A-A of FIG 5;
FIG. **7** is a diagram of a vertical pipe orifice shaped sealing opening on an elastic sealing rubber film;
FIG **8** is a diagram of a drainage tube and a two-way connector mechanism for two drainage tubes;
FIG **9** is diagram of four drainage tubes and a four-way connector mechanism which are arranged in a porous foam soft pad respectively;
FIG **10** is an overall structure diagram of an orthopedic vacuum sealing drainage device;
FIG **11** is a longitudinal sectional view of FIG **10****;**
FIG **12** is an overall structure diagram of a vacuum sealing drainage device used at the perineum; and
FIG **13** is a structure diagram of an anus inserting catheter.

### DETAILED DESCRIPTION OF THE EMBODIMENTS

Detailed description of the invention will be given below in conjunction with accompanying drawings.

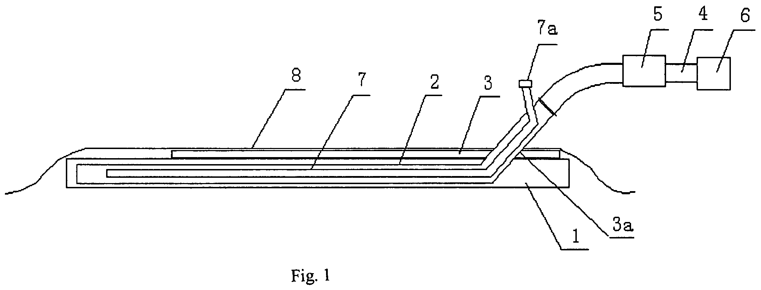

FIG. **1** is an overall structure diagram of a first basic vacuum sealing drainage device, which comprises:
a porous foam soft pad **1,** a drainage tube **2** arranged in the porous foam soft pad **1** and provided with a tail end extending outside the porous foam soft pad **1,** and an elastic sealing rubber film 3 for allowing tubes to extend out is arranged on the outer side of the porous foam soft pad **1.** A sealing opening **3a** for allowing the drainage tube **2** to extend out is a circular hole and formed on the elastic sealing rubber film **3.**

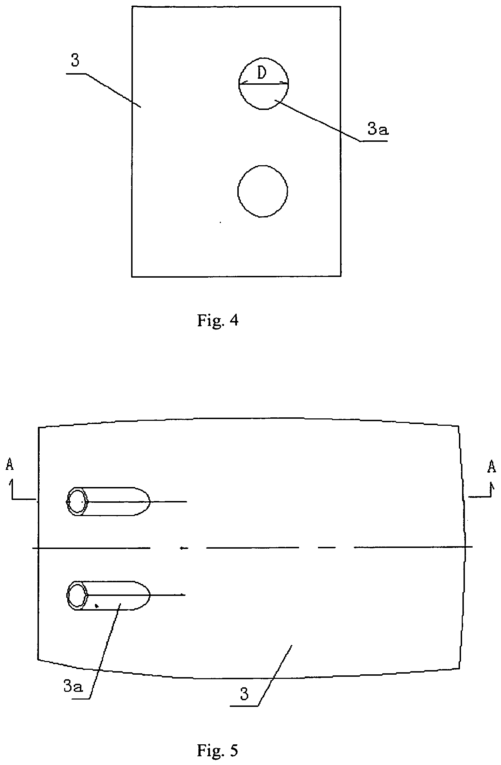

FIG **4** is a diagram of the elastic sealing rubber film. The diameter D of the sealing opening **3a** is 0.3-4 mm less than the outer diameter of the drainage tube **2.**

Due to the elasticity of the elastic sealing rubber film, the sealing opening **3a** is tightened on the drainage tube **2** for sealing. During the using process, a sealing film **8** is resealed on the elastic sealing rubber film **3** and the porous foam soft pad **1.** The tail end of the drainage tube **2** is connected with a drainage container **5** and a vacuum source **6** through a catheter **4.** The tail end of the drainage tube **2** extends out from the upper side of the porous foam soft pad **1.**

A flushing pipe **7** is arranged in the drainage tube **2,** one end of the flushing pipe **7** extends to the inside of the drainage tube **2,** the other end of the flushing pipe **7** is led out from the side wall at the tail end of the drainage tube **2,** and a sealing cover **7a** is arranged at the tail end of the flushing pipe **7.**

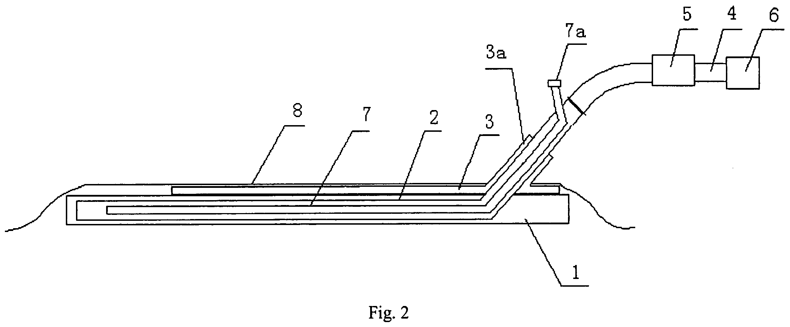

FIG **2** is an overall structure diagram of a second basic vacuum sealing drainage device. The second basic vacuum sealing drainage device has the same basic structure as the first basic vacuum sealing drainage device, however, the sealing opening **3a** used for allowing the drainage tube **2** to extend out and formed on the elastic sealing rubber film 3 is a pipe orifice, and the diameter of the opening part of the pipe orifice is 0.3-2 mm less than the outer diameter of a corresponding extending pipe.

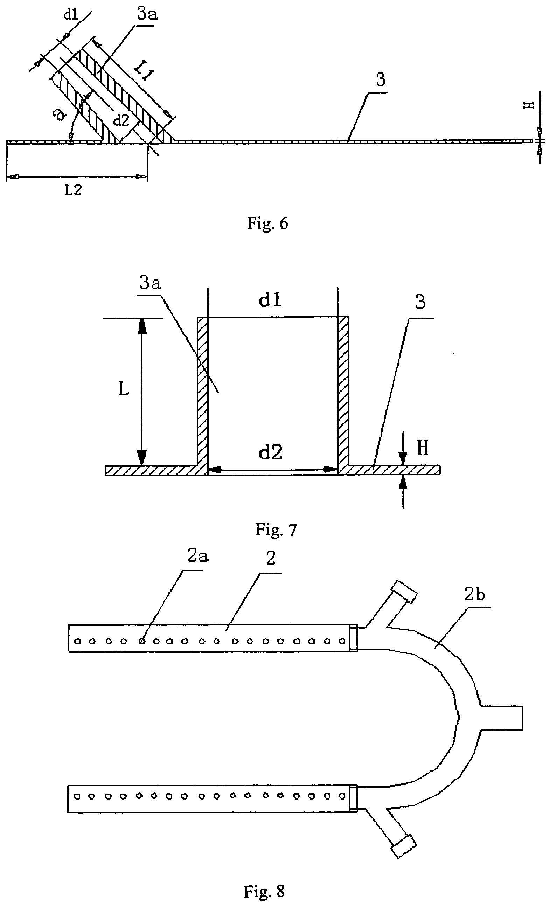

As shown in FIG. **5** and FIG. 6, the sealing opening **3a** used for allowing tubes to extend out and formed on the elastic sealing rubber film **3** is a pipe orifice, the pipe orifice takes the shape of an inverted horn, the diameter d1 of the opening part of the pipe orifice is 0.3-2 mm less than the outer diameter of the drainage tube **2,** and the diameter d2 of the bottom of the pipe orifice is 2-6 mm more than the outer diameter of the drainage tube **2.** The included angle α between the sealing opening **3a** and a main body of the elastic sealing rubber film **3** is 15-90°, and preferably 30-45°.

FIG. **7** is a diagram of a vertical pipe orifice shaped sealing opening on the elastic sealing rubber film. The included angle α between the sealing opening **3a** and the main body of the elastic sealing rubber film **3** is 90°. The embodiment is suitable for vertical tubes.

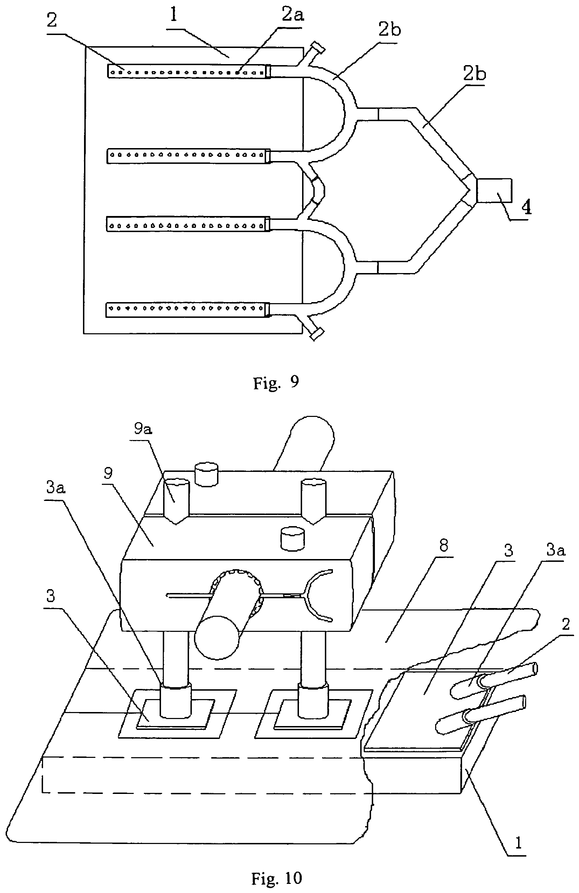

FIG. **8** is diagram of the drainage tube and a two-way connector mechanism for two drainage tubes. FIG **9** is a diagram of four drainage tubes and a four-way connector mechanism which are arranged in the porous foam soft pad respectively.

A plurality of small holes **2a** is formed on the side wall of the drainage tube **2.** A plurality of drainage tubes are connected with a multi-way connector **2b,** and the multi-way connector **2b** is connected with the catheter **4.** The drainage tubes are suitable for massive trauma. The plurality of the drainage tubes **2** are connected through the multi-way connector and then converged to enter the drainage container **5,** and the operation is simple and convenient. For the use inside the body, a tube is lead out, thus the wound is small.

The structure solves the problem of sealing, thus the stable vacuum sealing drainage can be kept.

When the medical vacuum sealing drainage device is used for wounds on the body surface, as shown in FIG. **1** and FIG **2****,** after the porous foam soft pad **1** is placed on the wound, the elastic sealing rubber film **3** is arranged at the position where the drainage tubes **2** extends out, and the sealing opening **3a** is tightened on the drainage tubes **2** for sealing due to the elasticity of the elastic sealing rubber film, thus the elastic sealing rubber film has the sealing effect. The sealing film **8** covers the porous foam soft pad **1** and the elastic sealing rubber film **3** for sealing, so that the porous foam soft pad **1** is sealed with the body surface. Besides achieving the sealing effect, the elastic sealing rubber film covers the porous foam soft pad by increasing the area of the elastic sealing rubber film **3,** so that the problem that if the sealing film **8** is directly stuck onto the porous foam soft pad **1,** the porous foam soft pad may become hard due to the evaporation of water in the porous foam soft pad through the sealing film, and then the vacuum sealing drainage will fail is avoided.

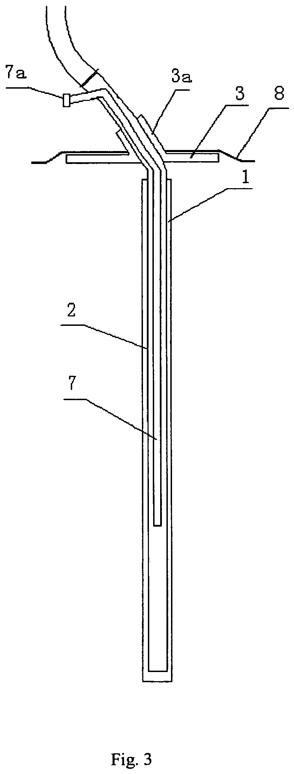

FIG **3** is a diagram of the medical vacuum sealing drainage device used inside the body.

The example adopts the structure form that one end of the drainage tubes **2** is positioned in the porous foam soft pad **1,** and the other end of the drainage tubes **2** is led out from the upper end of the porous foam soft pad **1.** When the medical vacuum sealing drainage device is used in the body cavity, after the porous foam soft pad **1** is placed in the body cavity, the elastic sealing rubber film **3** is attached outside the body to provide an effective support (carrier) for the sealing film **8,** and the sealing opening **3a** is tightened on the drainage tube **2** for sealing due to the elasticity of the elastic sealing rubber film, thus the sealing effect is achieved. The sealing film **8** covers the elastic sealing rubber film **3,** and the periphery of the elastic sealing rubber film **3** is sealed with the body surface.

When the small holes of the drainage tube **2** are blocked, a vacuum system is closed, the sealing cover **7a** at the tail end of the flushing pipe **7** is opened, an irrigator is accessed, and normal saline is injected, so that stagnant blood clots are diluted and dissolved. After stopping washing, the vacuum system is opened, and the diluted stagnant blood clots are drawn out.

The current commonly-used washing method is as follows: (1) a rubber hose is additionally arranged at the wound, but the lead-out position of the rubber hose is also required to be sealed by a film sticking method, thus the possibility of air leakage and the sealing difficulty are increased; (2) when flushing fluid is retrogradely injected from the original drainage tube, the drained exudate may be reinjected to the wound to lead to retrograde infection; (3) when flushing fluid is injected through holes perforated on the porous foam soft pad, the sealing film is only required to be restuck at the perforated hole on the porous foam soft pad, thus the operating difficulty is increased.

In the medical vacuum sealing drainage device, the blocked pipe is conveniently flushed, the phenomenon of retrograde infection is avoided, and the flushing pipe does not occupy the space of the drainage tube. The sealing cover **7a** is arranged at the tail end of the flushing pipe **7,** and when the flushing pipe **7** is not used, the sealing cover is closed, so as to ensure the normal work of the drainage tube **2.** The flushing pipe **7** also can be used as a sampling opening, and the drainage fluid is tested as the reference for medication for doctors.

A plurality of drainage tubes **2** are connected through a multi-way connector, when one drainage tube is blocked, observation can be carried out conveniently from a catheter connector, so that the blocked drainage tube is conveniently flushed in time through a flushing pipe, other drainage tubes can be continued to conduct drainage, and the phenomenon that the vacuum sealing drainage device cannot work normally after one drainage tube is blocked is avoided.

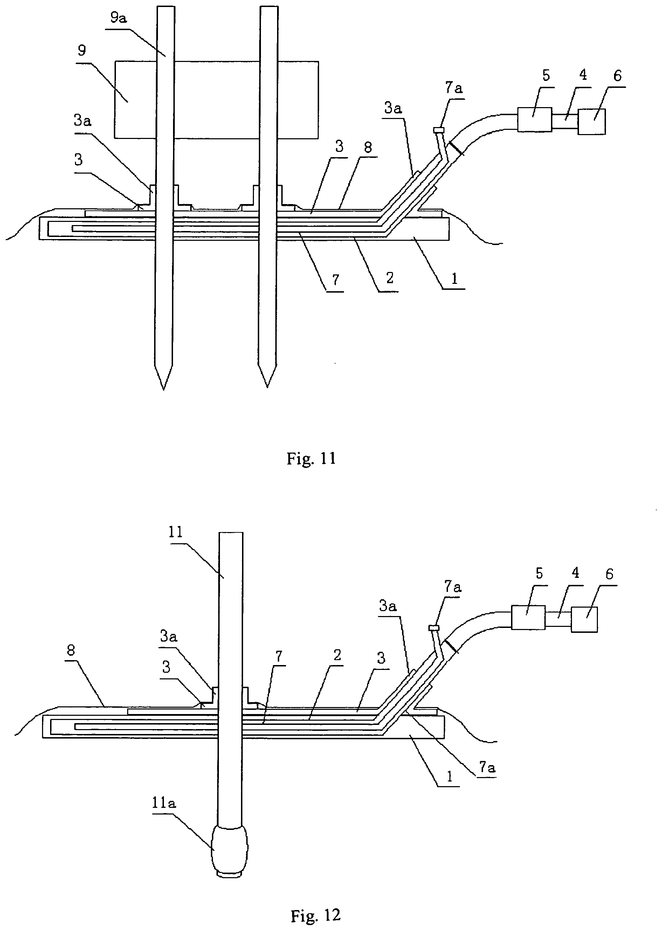

FIG **10** and FIG. **11** are overall structure diagrams of orthopedic vacuum sealing drainage devices.

The orthopaedic vacuum sealing drainage device has the basically same structure as the basic vacuum sealing drainage devices, and comprises a porous foam soft pad **1,** and a drainage tube **2** arranged in the porous foam soft pad **1** and provided with a tail end extending outside the porous foam soft pad **1,** a first elastic sealing rubber film **3** is arranged on the outer side of the porous foam soft pad **1,** a sealing opening **3a** for allowing the drainage tube **2** to extend out is formed on the first elastic sealing rubber film **3,** the diameter D of the opening part of the sealing opening **3a** is smaller than the outer diameter of the corresponding drainage tube **2,** a second elastic sealing rubber film **3** and a third elastic sealing rubber film **3** are arranged at puncture needles **9a** connected with an orthopaedic external fixation device **9,** vertical pipe orifice shaped sealing openings 3a are formed on the second elastic sealing rubber film **3** and the third elastic sealing rubber film **3,** and sleeved on the two puncture needles **9a** respectively, and the second and the third elastic sealing rubber films **3** are placed on the first elastic sealing rubber film **3** or directly placed on the porous foam soft pad **1** for sealing in a covering manner. A flushing pipe **7** is arranged in the drainage tube **2,** one end of the flushing pipe **7** extends to the inside of the drainage tube **2,** the other end of the flushing pipe **7** is led out from the side wall at the tail end of the drainage tube **2,** and a sealing cover **7a** is arranged at the tail end of the sealing cover **7a.** The second and the third elastic sealing rubber films **3** are used to adapt the distance change of the two puncture needles **9a.** When the sealing opening **3a** used for allowing the drainage tube **2** to extend out and formed on the first elastic sealing rubber films **3** is a pipe orifice, the included angle α between the pipe orifice and a main body of the elastic sealing rubber film **3** is 15-90°, and preferably 30-45°.

The pipe orifice shaped sealing opening **3a** preferably takes the shape of an inverted horn. The diameter d1 of the opening part of the pipe orifice is 1-2 mm less than the outer diameter of the drainage tube **2,** and the diameter d2 of the bottom of the pipe orifice is 2-6 mm more than the outer diameter of the drainage tube **2.**

The sealing openings **3a** on the second and the third elastic sealing rubber films **3** are vertical pipe orifice shaped sealing openings **3a** shown in FIG. **7****,** belong to the embodiment that the included angle α between the pipe orifice and the main body of the elastic sealing rubber films **3** is 90°, and used for sealing other tubes. The pipe orifice shaped sealing opening **3a** takes the shape of an inverted horn, the diameter d1 of the opening part of the pipe orifice is 1-2 mm less than the outer diameter of the puncture needles **9a,** and the diameter d2 of the bottom of the pipe orifice is 2-6 mm more than the outer diameter of the corresponding puncture needles **9a.** The orthopaedic vacuum sealing drainage device changes the operating instructions and rules of the traditional orthopaedic external fixation technology and combines the sealing and fixing operation, and the orthopaedic external fixation device is hermetically sealed, so that the problem of failure or reduced effect in vacuum sealing drainage treatment due to air leakage is eliminated.

FIG **12** is an overall structure diagram of a vacuum sealing drainage device used at the perineum.

The vacuum sealing drainage device used at the perineum has the basically same structure as the basic vacuum sealing drainage devices, and comprises a porous foam soft pad **1,** and a drainage tube **2** arranged in the porous foam soft pad **1** and provided with a tail end extending outside the porous foam soft pad **1,** a first elastic sealing rubber film **3** is arranged on the outer side of the porous foam soft pad **1,** a sealing opening **3a** for allowing the drainage tube **2** to extend out is formed on the first elastic sealing rubber film **3,** and the diameter of the opening part of the sealing opening **3a** is smaller than the outer diameter of the corresponding drainage tube **2.** The vacuum sealing drainage device used at the perineum further comprises an anus inserting catheter **11,** a fourth elastic sealing rubber film **3** is arranged at the position where the anus inserting catheter **11** penetrates a vacuum area of the vacuum sealing drainage device, and provided with a vertical pipe orifice shaped sealing opening **3a,** the vertical pipe orifice shaped sealing opening **3a** is sleeved on the anus inserting catheter **11,** and the fourth elastic sealing rubber film **3** is placed on the first elastic sealing rubber film **3** or directly placed on the porous foam soft pad **1** for sealing in a covering manner. A flushing pipe **7** is arranged in the drainage tube **2,** one end of the flushing pipe **7** extends to the inside of the drainage tube **2,** the other end of the flushing pipe **7** is led out from the side wall at the tail end of the drainage tube **2,** and a sealing cover **7a** is arranged at the tail end of the flushing pipe **7.**

The pipe orifice shaped sealing opening **3a** of the fourth elastic sealing rubber film **3** preferably takes the shape of an inverted horn, the diameter d1 of the opening part of the pipe orifice is less than 1-2 mm less than the outer diameter of the anus inserting catheter **11,** and the diameter d2 of the bottom of the pipe orifice is 2-6 mm more than the corresponding anus inserting catheter **11.**

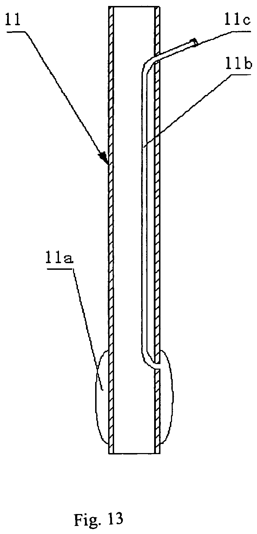

As shown in FIG **13****,** an annular rubber air-filled or liquid-filled bag **11a** is arranged on the outer side at the front end of the anus inserting catheter **11,** an air or liquid guide pipe **11b** parallel to the anus inserting catheter **11** is also arranged, the front end of the air or liquid guide pipe **11b** is communicated with the rubber air-filled or liquid-filled bag **11a,** the rear end of the air or liquid guide pipe **11b** extends outside the rear end of the anus inserting catheter **11,** and a sealing cover **11c** is arranged at the rear end of the air or liquid guide pipe **11b.** The liquid guide pipe **11b** also can be arranged on the inner wall of the anus inserting catheter **11.** In the VSD (vacuum sealing drainage) treatment, the anus inserting catheter is additionally arranged, so that the normal physiological excretion of patients is not affected, the excrement is effectively prevented from overflowing to pollute the wound, the incidence rate of infection is effectively lowered, and the wound healing time of patients is reduced.

In conclusion, skillfully solving the difficult problem (air leakage) unsolved for a long time through sealing the puncture tube part of the vacuum sealing drainage device under the elasticity of an elastic material is the core of the vacuum sealing drainage device. In a set of the vacuum sealing drainage devices, one or more can be used as required, and the vacuum sealing drainage devices with different sizes and shapes can be used alternately, thus the vacuum sealing drainage device is extremely flexible to use.

## Claims

1. A medical vacuum sealing drainage device, comprising:
a porous foam soft pad (1); and
a drainage tube (2) arranged in the porous foam soft pad (1) and provided with a tail end extending outside the porous foam soft pad (1);
wherein an elastic sealant film (3) is arranged on the outer side of the porous foam soft pad (1);
a sealing opening (3a) for allowing the drainage tube (2) to extend out is formed on the elastic sealant film (3);
**characterized in that** the diameter of the opening part of the sealing opening (3a) is less than the outer diameter of the drainage tube (2); and
a flushing pipe (7) is arranged in the drainage tube (2), one end of the flushing pipe (7) extends to the inside of the drainage tube (2), the other end of the flushing pipe (7) is led out from the side wall at the tail end of the drainage tube (2), and a sealing cover (7a) is arranged at the tail end of the flushing pipe (7).

2. The device of claim 1, **characterized in that** the sealing opening (3a) used for allowing the drainage tube (2) to extend out and formed on the elastic sealant film (3) is a circle hole, the diameter of which is 0.3-4 mm less than the outer diameter of the drainage tube (2).

3. The device of claim 1, **characterized in that** the sealing opening (3a) used for allowing the drainage tube (2) to extend out and formed on the elastic sealant film (3) is a pipe orifice, and the diameter of the opening part of the pipe orifice is 0.3-2 mm less than the outer diameter of the drainage tube (2).

4. The device of claim 3, **characterized in that** the sealing opening (3a) used for allowing tubes to extend out and formed on the elastic sealant film (3) is a pipe orifice, the pipe orifice takes a shape of an inverted horn, the diameter of the opening part of the pipe orifice is 1-2 mm less than the outer diameter of the drainage tube (2), and the diameter of the bottom of the pipe orifice is 2-6 mm more than the outer diameter of the drainage tube (2).

5. The device of claim 3 or 4, **characterized in that** the sealing opening (3a) used for allowing tubes to extend out and formed on the elastic sealant film (3) is a pipe orifice, an included angle between the sealing opening (3a) and a main body of the elastic sealant film (3) is 15-90°.

6. The device of claim 1 or 2, **characterized in that** a plurality of small holes (2a) are formed on the side wall of the drainage tube (2).

7. The device of claim 3 or 4, **characterized in that** a plurality of small holes (2a) are formed on the side wall of the drainage tube (2).

8. The device of claim 1 or 2, **characterized in that** a single or a plurality of drainage tubes (2) are employed; multiple drainage tubes (2) are arranged evenly with a distance of 25-35 mm between each other, and are connected through a multi-way connector.

9. The device of claim 3 or 4, **characterized in that** a single or a plurality of drainage tubes (2) are employed; multiple drainage tubes (2) are arranged evenly with a distance of 25-35 mm between each other, and are connected through a multi-way connector.

10. An orthopedic vacuum sealing drainage device, comprising:
a porous foam soft pad (1), and
a drainage tube (2) arranged in the porous foam soft pad (1) and provided with a tail end extending outside the porous foam soft pad (1);
wherein a first elastic sealant film (3) is arranged on the outer side of the porous foam soft pad (1), a sealing opening (3a) for allowing the drainage tube (2) to extend out is formed on the first elastic sealant film (3), the diameter of the opening part of the sealing opening (3a) is smaller than the outer diameter of the corresponding drainage tube (2);
a second elastic sealant film (3) and a third elastic sealant film (3) are arranged at puncture needles (9a) connected with an orthopaedic external fixation device (9), vertical pipe orifice shaped sealing openings (3a) are formed on the second elastic sealant film (3) and the third elastic sealant film (3), and sleeved on the two puncture needles (9a) respectively;
the second and the third elastic sealant film (3) are placed on the first elastic sealant film (3) or directly placed on the porous foam soft pad (1) for sealing in a covering manner; and
a flushing pipe (7) is arranged in the drainage tube (2), one end of the flushing pipe (7) extends to the inside of the drainage tube (2), the other end of the flushing pipe (7) is led out from the side wall at the tail end of the drainage tube (2), and a sealing cover (7a) is arranged at the tail end of the flushing pipe (7).

11. The device of claim 10, wherein the sealing opening (3a) used for allowing the drainage tube (2) to extend out and formed on the first elastic sealant films (3) is a pipe orifice, an included angle between the pipe orifice and a main body of the elastic sealant film (3) is 15-90°.

12. The device of claim 10 or 11, wherein the vertical pipe orifice shaped sealing opening on the second and the third elastic sealant film (3) takes a shape of an inverted horn, the diameter of the opening part of the pipe orifice is 0.3-2 mm less than the outer diameter of the puncture needles (9a), and the diameter of the bottom of the pipe orifice is 2-6 mm more than the outer diameter of the corresponding puncture needles (9a).

13. A vacuum sealing drainage device used at the perineum, comprising
a porous foam soft pad (1), and
a drainage tube (2) arranged in the porous foam soft pad (1) and provided with a tail end extending outside the porous foam soft pad (1);
wherein a first elastic sealant film (3) is arranged on the outer side of the porous foam soft pad (1), a sealing opening (3a) for allowing the drainage tube (2) to extend out is formed on the first elastic sealant film (3), and the diameter of the opening part of the sealing opening (3a) is smaller than the outer diameter of the corresponding drainage tube (2);
the vacuum sealing drainage device used at the perineum further comprises an anus inserting catheter (11), a further elastic sealant film (3) is arranged at the position where the anus inserting catheter (11) penetrates a vacuum area of the vacuum sealing drainage device, and provided with a vertical pipe orifice shaped sealing opening (3a), the vertical pipe orifice shaped sealing opening (3a) is sleeved on the anus inserting catheter (11), and the further elastic sealant film (3) is placed on the first elastic sealant film (3) or directly placed on the porous foam soft pad (1) for sealing in a covering manner; and
a flushing pipe (7) is arranged in the drainage tube (2), one end of the flushing pipe (7) extends to the inside of the drainage tube (2), the other end of the flushing pipe (7) is led out from the side wall at the tail end of the drainage tube (2), and a sealing cover (7a) is arranged at the tail end of the flushing pipe (7).

14. The device of claim 13, wherein
an annular rubber air-filled or liquid-filled bag (11a) is arranged on the outer side at the front end of the anus inserting catheter (11);
an air or liquid guide pipe (11b) parallel to the anus inserting catheter (11) is also arranged; and
the front end of the air or liquid guide pipe (11b) is communicated with the rubber air-filled or liquid-filled bag (11 a), the rear end of the air or liquid guide pipe (11b) extends outside the rear end of the anus inserting catheter (11), and a sealing cover (11c) is arranged at the rear end of the air or liquid guide pipe (11b).

## Patentansprüche

1. Medizinische Drainagevorrichtung für Vakuumversiegelung, umfassend:
ein Softpad (1) aus porösem Schaumstoff; und
einen Drainageschlauch (2), der in dem Softpad (1) aus porösem Schaumstoff angeordnet ist und mit einem Endstück versehen ist, das sich nach außerhalb des Softpads (1) aus porösem Schaumstoff erstreckt;
wobei
eine elastische Versiegelungsfolie (3) auf der Außenseite des Softpads (1) aus porösem Schaumstoff angeordnet ist;
eine Versiegelungsöffnung (3a) auf der elastischen Versiegelungsfolie (3) ausgebildet ist, die es dem Drainageschlauch (2) erlaubt, sich nach außen zu erstrecken;
**dadurch gekennzeichnet, dass**
der Durchmesser des Öffnungsteils der Versiegelungsöffnung (3a) kleiner als der Außendurchmesser des Drainageschlauchs (2) ist; und
ein Spülrohr (7) in dem Drainageschlauch (2) angeordnet ist, sich ein Ende des Spülrohrs (7) bis in das Innere des Drainageschlauchs (2) erstreckt, das andere Ende des Spülrohrs (7) aus der Seitenwand an dem Endstück des Drainageschlauchs (2) herausgeführt wird, und eine Versiegelungsabdeckung (7a) an dem Endstück des Spülrohrs (7) angeordnet ist.

2. Vorrichtung nach Anspruch 1, **dadurch gekennzeichnet, dass** die Versiegelungsöffnung (3a), die verwendet wird, damit sich der Drainageschlauch (2) nach außen erstrecken kann, und die auf der elastischen Versiegelungsfolie (3) ausgebildet ist, ein Kreisloch ist, dessen Durchmesser um 0,3 bis 4 mm kleiner als der Außendurchmesser des Drainageschlauchs (2) ist.

3. Vorrichtung nach Anspruch 1, **dadurch gekennzeichnet, dass** die Versiegelungsöffnung (3a), die verwendet wird, damit sich der Drainageschlauch (2) nach außen erstrecken kann, und die auf der elastischen Versiegelungsfolie (3) ausgebildet ist, eine Rohrmündung ist, und der Durchmesser des Öffnungsteils der Rohrmündung um 0,3 bis 2 mm kleiner als der Außendurchmesser des Drainageschlauchs (2) ist.

4. Vorrichtung nach Anspruch 3, **dadurch gekennzeichnet, dass** die Versiegelungsöffnung (3a), die verwendet wird, damit sich Schläuche nach außen erstrecken können, und die auf der elastischen Versiegelungsfolie (3) ausgebildet ist, eine Rohrmündung ist, wobei die Rohrmündung die Form eines umgekehrten Trichters annimmt, wobei der Durchmesser des Öffnungsteils der Rohrmündung um 1 bis 2 mm kleiner als der Außendurchmesser des Drainageschlauchs (2) ist, und der Durchmesser des Bodens der Rohrmündung um 2 bis 6 mm größer als der Außendurchmesser des Drainageschlauchs (2) ist.

5. Vorrichtung nach Anspruch 3 oder 4, **dadurch gekennzeichnet, dass** die Versiegelungsöffnung (3a) die verwendet wird, damit sich Schläuche nach außen erstrecken können, und die auf der elastischen Versiegelungsfolie (3) ausgebildet ist, eine Rohrmündung ist, wobei ein eingeschlossener Winkel zwischen der Versiegelungsöffnung (3a) und einem Hauptkörper der elastischen Versiegelungsfolie (3) 15 bis 90° beträgt.

6. Vorrichtung nach Anspruch 1 oder 2, **dadurch gekennzeichnet, dass** eine Vielzahl von kleinen Löchern (2a) an der Seitenwand des Drainageschlauchs (2) ausgebildet ist.

7. Vorrichtung nach Anspruch 3 oder 4, **dadurch gekennzeichnet, dass** eine Vielzahl von kleinen Löchern (2a) an der Seitenwand des Drainageschlauchs (2) ausgebildet ist.

8. Vorrichtung nach Anspruch 1 oder 2, **dadurch gekennzeichnet, dass** ein einziger oder eine Vielzahl von Drainageschläuchen (2) verwendet wird; mehrere Drainageschläuche (2) mit einem Abstand von 25 bis 35 mm dazwischen gleichmäßig angeordnet sind und über einen Vielfachstecker verbunden sind.

9. Vorrichtung nach Anspruch 3 oder 4, **dadurch gekennzeichnet, dass** ein einziger oder eine Vielzahl von Drainageschläuchen (2) verwendet wird; mehrere Drainageschläuche (2) mit einem Abstand von 25 bis 35 mm dazwischen gleichmäßig angeordnet sind und über einen Vielfachstecker verbunden sind.

10. Orthopädische Drainagevorrichtung für Vakuumversiegelung, umfassend:
ein Softpad (1) aus porösem Schaumstoff, und
einen Drainageschlauch (2), der in dem Softpad (1) aus porösem Schaumstoff angeordnet ist und mit einem Endstück versehen ist, das sich nach außerhalb des Softpads (1) aus porösem Schaumstoff erstreckt;
wobei
eine erste elastische Versiegelungsfolie (3) auf der Außenseite des Softpads (1) aus porösem Schaumstoff angeordnet ist, eine Versiegelungsöffnung (3a) auf der ersten elastischen Versiegelungsfolie (3) ausgebildet ist, die es dem Drainageschlauch (2) erlaubt, sich nach außen zu erstrecken, wobei der Durchmesser des Öffnungsteils der Versiegelungsöffnung (3a) kleiner als der Außendurchmesser des entsprechenden Drainageschlauchs (2) ist;
eine zweite elastische Versiegelungsfolie (3) und eine dritte elastische Versiegelungsfolie (3) an Einstechkanülen (9a) angeordnet sind, die mit einer orthopädischen externen Befestigungsvorrichtung (9) verbunden sind, senkrechte, als Rohrmündung gestaltete Versiegelungsöffnungen (3a) auf der zweiten elastischen Versiegelungsfolie (3) und der dritten elastischen Versiegelungsfolie (3) ausgebildet sind, und jeweils auf die zwei Einstechkanülen (9a) aufgeschoben werden;
die zweite und die dritte elastische Versiegelungsfolie (3) auf der ersten elastischen Versiegelungsfolie (3) platziert sind oder direkt auf dem Softpad (1) aus porösem Schaumstoff für eine abdeckende Versiegelung platziert sind; und
ein Spülrohr (7) in dem Drainageschlauch (2) angeordnet ist, sich ein Ende des Spülrohrs (7) bis in das Innern des Drainageschlauchs (2) erstreckt, das andere Ende des Spülrohrs (7) aus der Seitenwand heraus an dem Endstück des Drainageschlauchs (2) geführt wird, und eine Versiegelungsabdeckung (7a) an dem Endstück des Spülrohrs (7) angeordnet ist.

11. Vorrichtung nach Anspruch 10, wobei die Versiegelungsöffnung (3a), die verwendet wird, damit sich der Drainageschlauch (2) nach außen erstrecken kann, und die auf der ersten elastischen Versiegelungsfolie (3) ausgebildet ist, eine Rohrmündung ist, wobei ein eingeschlossener Winkel zwischen der Rohrmündung und einem Hauptkörper der elastischen Versiegelungsfolie (3) 15 bis 90° beträgt.

12. Vorrichtung nach Anspruch 10 oder 11, wobei die senkrechte, als Rohrmündung gestaltete Versiegelungsöffnung auf der zweiten und der dritten elastischen Versiegelungsfolie (3) die Form eines umgekehrten Trichters annimmt, der Durchmesser des Öffnungsteils der Rohrmündung um 0,3 bis 2 mm kleiner als der Außendurchmesser der Einstechkanülen (9a) ist, und der Durchmesser des Bodens der Rohrmündung um 2 bis 6 mm größer als der Außendurchmesser der entsprechenden Einstechkanülen (9a) ist.

13. Drainagevorrichtung für Vakuumversiegelung, die am Perineum verwendet wird, umfassend
ein Softpad (1) aus porösem Schaumstoff, und
einen Drainageschlauch (2), der in dem Softpad (1) aus porösem Schaumstoff angeordnet ist und mit einem Endstück versehen ist, das sich aus dem Softpad (1) aus porösem Schaumstoff nach außen erstreckt;
wobei
eine erste elastische Versiegelungsfolie (3) auf der Außenseite des Softpads (1) aus porösem Schaumstoff angeordnet ist, eine Versiegelungsöffnung (3a) auf der ersten elastischen Versiegelungsfolie (3) ausgebildet ist, die es dem Drainageschlauch (2) erlaubt, sich nach außen zu erstrecken, und der Durchmesser des Öffnungsteils der Versiegelungsöffnung (3a) kleiner als der Außendurchmesser des entsprechenden Drainageschlauchs (2) ist;
die Drainagevorrichtung für Vakuumversiegelung, die am Perineum verwendet wird, ferner einen Rektalkatheter (11) umfasst, eine weitere elastische Versiegelungsfolie (3) in der Position angeordnet ist, in welcher der Rektalkatheter (11) in einen Vakuumbereich der Drainagevorrichtung für Vakuumversiegelung eindringt, und die mit einer senkrechten, als Rohrmündung gestalteten Versiegelungsöffnung (3a) versehen ist, die senkrechte, als Rohrmündung gestaltete Versiegelungsöffnung (3a) auf den Rektalkatheter (11) aufgeschoben wird, und die weitere elastische Versiegelungsfolie (3) auf die erste elastische Versiegelungsfolie (3) platziert wird oder direkt auf das Softpad (1) aus porösem Schaumstoff zur abdeckenden Versiegelung platziert wird; und
ein Spülrohr (7) in dem Drainageschlauch (2) angeordnet ist, sich ein Ende des Spülrohrs (7) in das Innere des Drainageschlauchs (2) erstreckt, das andere Ende des Spülrohrs (7) aus der Seitenwand an dem Endstück des Drainageschlauchs (2) herausgeführt wird, und eine Versiegelungsabdeckung (7a) an dem Endstück des Spülrohrs (7) angeordnet ist.

14. Vorrichtung nach Anspruch 13, wobei
ein ringförmiger, mit Luft oder Flüssigkeit gefüllter Gummibeutel (11a) auf der Außenseite an dem vorderen Endes des Rektalkatheters (11) angeordnet ist;
ein Rohr (11b) zum Führen von Luft oder Flüssigkeit parallel zum Rektalkatheter (11) ebenfalls angeordnet ist; und
das vordere Ende des Rohrs (11b) zum Führen von Luft oder Flüssigkeit mit dem mit Luft oder Flüssigkeit gefüllten Beutel (11a) in Verbindung gesetzt wird, sich das hintere Ende des Rohrs (11b) zum Führen von Luft oder Flüssigkeit außerhalb des hinteren Endes des Rektalkatheters (11) erstreckt, und eine Versiegelungsabdeckung (11c) am hinteren Ende des Rohrs (11b) zum Führen von Luft oder Flüssigkeit angeordnet ist.

## Revendications

1. Dispositif de drainage étanchéifiant sous vide médical, comprenant :
un tampon souple poreux en mousse (1) ; et
un tube de drainage (2) agencé dans le tampon souple poreux en mousse (1) et doté d'une extrémité de queue s'étendant hors du tampon souple poreux en mousse (1) ;
dans lequel
un film de matériau d'étanchéité élastique (3) est agencé sur le côté extérieur du tampon souple poreux en mousse (1) ;
une ouverture d'étanchéité (3a) pour permettre au tube de drainage (2) de s'étendre vers l'extérieur est formée sur le film de matériau d'étanchéité élastique (3) ;
**caractérisé en ce que**
le diamètre de la partie d'ouverture de l'ouverture d'étanchéité (3a) est inférieur au diamètre extérieur du tube de drainage (2) ; et
un tuyau de rinçage (7) est agencé dans le tube de drainage (2), une extrémité du tuyau de drainage (7) s'étend vers l'intérieur du tube de drainage (2), l'autre extrémité du tuyau de rinçage (7) est dirigée hors de la paroi latérale à l'extrémité de queue du tube de drainage (2) et un capot d'étanchéité (7a) est agencé à l'extrémité de queue du tuyau de rinçage (7).

2. Dispositif selon la revendication 1, **caractérisé en ce que** l'ouverture d'étanchéité (3a) utilisée pour permettre au tube de drainage (2) de s'étendre vers l'extérieur et formée sur le film de matériau d'étanchéité élastique (3) est un trou circulaire dont le diamètre est 0,3-4 mm inférieur au diamètre extérieur du tube de drainage (2).

3. Dispositif selon la revendication 1, **caractérisé en ce que** l'ouverture d'étanchéité (3a) utilisée pour permettre au tube de drainage (2) de s'étendre vers l'extérieur et formée sur le film de matériau d'étanchéité élastique (3) est un orifice de tuyau et le diamètre de la partie d'ouverture de l'orifice de tuyau est de 0,3-2 mm inférieur au diamètre extérieur du tube de drainage (2).

4. Dispositif selon la revendication 3, **caractérisé en ce que** l'ouverture d'étanchéité (3a) utilisée pour permettre aux tubes de s'étendre vers l'extérieur et formée sur le film de matériau d'étanchéité élastique (3) est un orifice de tuyau, l'orifice de tuyau a la forme d'un cornet inversé, le diamètre de la partie d'ouverture de l'orifice de tuyau est de 1-2 mm inférieur au diamètre extérieur du tube de drainage (2), et le diamètre du fond de l'orifice de tuyau est de 2-6 mm supérieur au diamètre extérieur du tube de drainage (2).

5. Dispositif selon la revendication 3 ou 4, **caractérisé en ce que** l'ouverture d'étanchéité (3a) utilisée pour permettre aux tubes de s'étendre vers l'extérieur et formée sur le film de matériau d'étanchéité élastique (3) est un orifice de tuyau, un angle inclus entre l'ouverture d'étanchéité (3a) et un corps principal du film de matériau d'étanchéité élastique (3) est de 15-90°.

6. Dispositif selon la revendication 1 ou 2, **caractérisé en ce qu'**une pluralité de petits trous (2a) sont formés sur la paroi latérale du tube de drainage (2).

7. Dispositif selon la revendication 3 ou 4, **caractérisé en ce qu'**une pluralité de petits trous (2a) sont formés sur la paroi latérale du tube de drainage (2).

8. Dispositif selon la revendication 1 ou 2, **caractérisé en ce qu'**un seul ou une pluralité tube(s) de drainage (2) est/sont employé(s) ; des tubes de drainage (2) multiples sont agencés de manière régulière à une distance de 25-35 mm entre eux et sont reliés par un connecteur multi-voies.

9. Dispositif selon la revendication 3 ou 4, **caractérisé en ce qu'**un seul ou une pluralité tube(s) de drainage (2) est/sont employé(s) ; des tubes de drainage (2) multiples sont agencés de manière régulière à une distance de 25-35 mm entre eux et sont reliés par un connecteur multi-voies.

10. Dispositif de drainage étanchéifiant sous vide orthopédique, comprenant :
un tampon souple poreux en mousse (1) ; et
un tube de drainage (2) agencé dans le tampon souple poreux en mousse (1) et doté d'une extrémité de queue s'étendant hors du tampon souple poreux en mousse (1) ;
dans lequel
un premier film de matériau d'étanchéité élastique (3) est agencé sur le côté extérieur du tampon souple poreux en mousse (1), une ouverture d'étanchéité (3a) pour permettre au tube de drainage (2) de s'étendre vers l'extérieur est formée sur le premier film de matériau d'étanchéité élastique (3), le diamètre de la partie d'ouverture de l'ouverture d'étanchéité (3a) est inférieur au diamètre extérieur du tube de drainage (2) correspondant ; et
un deuxième film de matériau d'étanchéité élastique (3) et un troisième film de matériau d'étanchéité élastique (3) sont agencés sur des aiguilles de perforation (9a) reliées à un dispositif de fixation externe orthopédique (9), des ouvertures d'étanchéité (3a) en forme d'orifice de tuyau vertical sont formées sur le deuxième film de matériau d'étanchéité élastique (3) et le troisième film de matériau d'étanchéité élastique (3) et manchonnées sur les deux aiguilles de perforation (9a) respectivement ;
les deuxième et troisième films de matériau d'étanchéité élastique (3) sont placés sur le premier film de matériau d'étanchéité élastique (3) ou placés directement sur le tampon souple poreux en mousse (1) pour étanchéifier en couvrant ; et
un tuyau de rinçage (7) est agencé dans le tube de drainage (2), une extrémité du tuyau de drainage (7) s'étend vers l'intérieur du tube de drainage (2) l'autre extrémité du tuyau de rinçage (7) est dirigée hors de la paroi latérale à l'extrémité de queue du tube de drainage (2) et un capot d'étanchéité (7a) est agencé à l'extrémité de queue du tuyau de rinçage (7).

11. Dispositif selon la revendication 10, dans lequel l'ouverture d'étanchéité (3a) utilisée pour permettre au tube de drainage (2) de s'étendre vers l'extérieur et formée sur le premier film de matériau d'étanchéité élastique (3) est un orifice de tuyau, un angle inclus entre l'orifice de tuyau et un corps principal du film de matériau d'étanchéité élastique (3) est de 15-90°.

12. Dispositif selon la revendication 10 ou 11, dans lequel l'ouverture d'étanchéité d'étanchéité en forme d'orifice de tuyau vertical sur le deuxième et le troisième film de matériau d'étanchéité élastique (3) a la forme d'un cornet inversé, le diamètre de la partie d'ouverture de l'orifice de tuyau est 0,3-2 mm inférieur au diamètre extérieur des aiguilles de perforation (9a), et le diamètre du fond de l'orifice de tuyau est 2-6 mm supérieur au diamètre extérieur des aiguilles de perforation (9a) correspondantes.

13. Dispositif de drainage étanchéifiant sous vide utilisé au niveau du périnée, comprenant :
un tampon souple poreux en mousse (1) ; et
un tube de drainage (2) agencé dans le tampon souple poreux en mousse (1) et doté d'une extrémité de queue s'étendant hors du tampon souple poreux en mousse (1) ;
dans lequel
un premier film de matériau d'étanchéité élastique (3) est agencé sur le côté extérieur du tampon souple poreux en mousse (1), une ouverture d'étanchéité (3a) pour permettre au tube de drainage (2) de s'étendre vers l'extérieur est formée sur le premier film de matériau d'étanchéité élastique (3) et le diamètre de la partie d'ouverture de l'ouverture d'étanchéité (3a) est inférieur au diamètre extérieur du tube de drainage (2) correspondant ;
le dispositif de drainage étanchéifiant sous vide utilisé au niveau du périnée comprend en outre un cathéter d'insertion anale (11), un film de matériau d'étanchéité élastique (3) supplémentaire est agencé à la position où le cathéter d'insertion anale (11) pénètre une zone sous vide du dispositif de drainage étanchéifiant sous vide, et doté d'une ouverture d'étanchéité (3a) en forme d'orifice de tuyau vertical, l'ouverture d'étanchéité (3a) en forme d'orifice de tuyau vertical est manchonnée sur le cathéter d'insertion anale (11), et le film de matériau d'étanchéité élastique (3) supplémentaire est placé sur le premier film de matériau d'étanchéité élastique (3) ou placé directement sur le tampon souple poreux en mousse (1) pour étanchéifier en couvrant ; et
un tuyau de rinçage (7) est agencé dans le tube de drainage (2), une extrémité du tuyau de drainage (7) s'étend vers l'intérieur du tube de drainage (2) l'autre extrémité du tuyau de rinçage (7) est dirigée hors de la paroi latérale à l'extrémité de queue du tube de drainage (2) et un capot d'étanchéité (7a) est agencé à l'extrémité de queue du tuyau de rinçage (7).

14. Dispositif selon la revendication 13, dans lequel
une poche en caoutchouc annulaire remplie de liquide ou remplie d'air (11a) est agencée sur un côté extérieur à l'extrémité avant du cathéter d'insertion anale (11) ;
un tuyau de guidage d'air ou de liquide (11b) parallèle au cathéter d'insertion anale (11) est également agencé ; et
l'extrémité avant du tuyau de guidage d'air ou de liquide (11b) communique avec la poche en caoutchouc annulaire remplie de liquide ou remplie d'air (11a), l'extrémité arrière du tuyau de guidage d'air ou de liquide (11b) s'étend à l'extérieur de l'extrémité arrière du cathéter d'insertion anale (11), et un capot d'étanchéité (11c) est agencé à l'extrémité arrière du tuyau de guidage d'air ou de liquide (11b).
